# EUROPEAN PATENT SPECIFICATION

(11) **EP 1 269 912 B1**
(45) Date of publication and mention of the grant of the patent: **28.04.2004**
(21) Application number: 02254361.5
(22) Date of filing: 21.06.2002
(51) Int. Cl.: A61B 5/05

(54) **A body fat monitoring device**
Körperfettüberwachungsgerät
Appareil de surveillance de la graisse corporelle

(30) Priority: 22.06.2001 GB 0115353
(43) Date of publication of application: 02.01.2003
(73) Proprietor: Terraillon Holdings Limited, Dublin (IE)
(72) Inventor: Stephens, Brian, Co. Dublin (IE)
(74) Representative: Johnson, Terence Leslie

(56) References cited:
- EP-A- 1 027 861
- EP-A- 1 080 686
- EP-A- 1 084 676
- EP-A- 1 095 613
- US-A- 5 886 302

## Description

The invention relates to a personal body fat monitoring device, and particularly to such a device incorporating a personal weighing scale.

Such prior art is exemplified by EP 1095613 A1.

According to the invention there is provided a personal body fat monitoring device, comprising at least one major surface element, electrical contacts exposed for contact by feet of a user, and electronic means for calculating body fat actuated by the contacts, characterised by the arrangement being such that when the electrical contacts are contacted, the body fat of a user is determined.

The major surface of the element may comprise a weight receiving platform of the scale. This provides a relatively simple construction.

The platform may comprise glass an upper in use major planar surface of which holds the contacts. This provides a relatively simple yet effective construction, particularly when the glass may comprise a sheet of glass *per se*.

Thus the major planar surface may be an upper, in use, surface of the platform.

The contacts may be disposed so as to provide a desired array whereby to be exposed and thus contactable by toe and heel regions of the feet of a user in use.

The glass may be a glass material. Herein the expression "glass material" includes glass or a glass-like material. In a preferred embodiment the weight-receiving element comprises glass *per se*, suitably transparent glass.

The device may extend to be also a personal weighing scale. This provides a composite obesity and weight monitoring device.

The contacts may be mechanically secured to a major surface of the platform, may be secured by an adhesive to a major surface of the platform, may be secured by electro-deposition or similar film contact to a major surface of the platform, or may be etched into a major surface of the platform. All these options provide a relative simple yet positive electrical contact with the user, whereby the body fat of a user may be monitored.

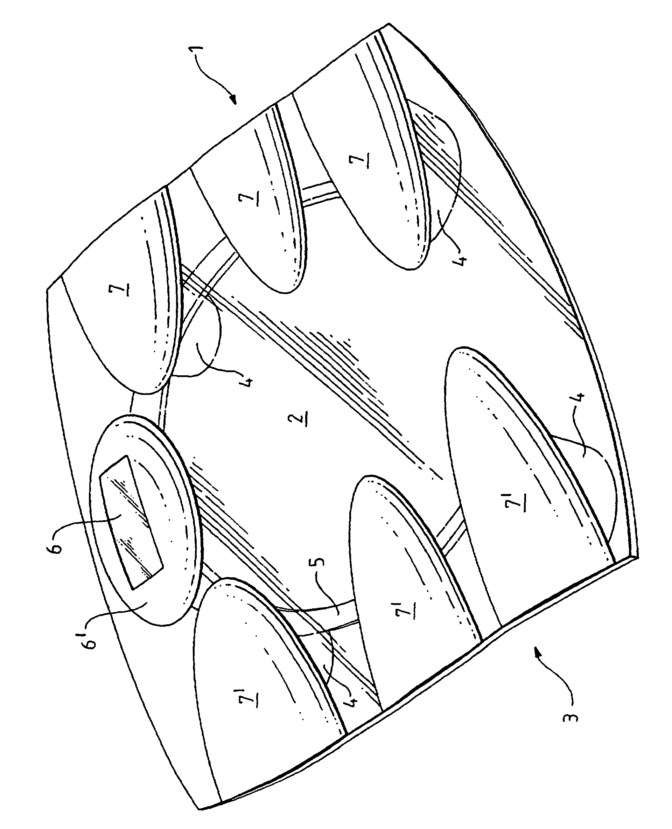

A personal weighing scale in the form of a body fat monitor is hereinafter described, by way of example, with reference to the accompanying drawing, which shows a perspective view of the scale from above.

Referring to the drawings there is shown a personal body fat monitoring device 1, comprising at least one major surface element 2 which comprises a glass material. In the embodiment the major surface element 2 is a weight receiving platform of the scale 1 which platform is made of transparent glass.

The platform 2 is supported on a base 3 comprising a load bearing mechanism in the embodiment having four feet 4 and an omega-shaped support 5. There is an LCD or LED read-out 6 and electronic means 6' comprising circuitry (not shown) and an array of electrical contacts 7 whereby when a user steps on the platform 2, placing his or her feet on the array of contacts to right and left 7 and 7' respectively (as viewed), the electronic means, which is programmed to provide a read-out of body fat of the person, is activated via the contacts 7,7' to provide that body fat read-out at the LCD or LED 6.

The contacts 7,7' are carried by the upper (in use) major surface of the weight receiving platform 2 being secured thereto or therein or thereon by any suitable means such as mechanically, by adhesive, by electro-deposition or etching.

It will be understood too that modifications are possible, for example the array of contacts 7,7' can be in any desired configuration other than the one shown, and the support may be a glass material body or base rather than the feet 4 and omega-shaped support 5 shown. Thus the body fat monitor may be a block of glass in, on or adjacent an upper surface in use on which a user stands and which carries the contacts 7,7'.

The configuration of the contacts 7,7' may be according to design and kw-impedance or capacitance requirements in providing body fat monitoring.

The glass platform is sufficiently strong to support the weight of a user.

Glass or a glass-like material as described and shown with reference to the drawing is preferred for the platform 2 at least as it can be readily formed to a desired shape and configuration as by cutting, so that no expensive moulding tool is required as is the case when plastic is used. This results in a lower production cost, and moreover glass is relatively easy to clean, easier than plastic, and is easy to maintain. Glass or glass material also provides a positive electrical connection of the contacts and electronic circuitry so as to provide a consistent monitoring of body fat of the user, and hence consistent read outs when the device is used regularly or sporadically over a period of time.

It will be understood in particular, that the contacts 7,7' are of metal and exposed in the upper major surface of the glass platform 2, and are so disposed in a desired array that the toe region and heel region of each foot of a user makes firm skin contact with the contacts in use. The contacts activate the electronic means which records from the weight of the person, the water content of the legs of that person and converts that data to a body fat reading, which is displayed on the LCD or LED 6, body fat being directly calculable from the said water content.

A device as shown in the drawing can also be used as a personal weighing scale, the electronics 6' being in addition to monitoring and calculating body fat, also being able to calculate, and display at the LCD 6, the weight of a user.

## Claims

1. A personal body fat monitoring device, comprising at least one major surface element (2), electrical contacts (7,7') exposed for contact by feet of a user, and electronic means (6') for calculating body fat actuated by the contacts (7,7'), **characterised by** the arrangement being such that when the electrical contacts are contacted, the electronic means is activated, so that the body fat of a user is determined.

2. A device according to Claim 1, **characterised by** the major surface element (2) comprising a weight receiving platform of the device (1).

3. A device according to Claim 2, **characterised by** the platform (2) comprising glass an upper in use major planar surface of which holds the contacts (7,7').

4. A device according to Claim 3, **characterised by** the glass comprising a sheet of glass (2) *per se*.

5. A device according to Claim 4, **characterised by** the contacts (7,7') being mechanically secured to the major surface of the glass platform.

6. A device according to Claim 4, **characterised by** the contacts (7,7') being secured by an adhesive to the major surface of the glass platform (2).

7. A device according to Claim 4, **characterised by** the contacts (7,7') being secured by electro-deposition or similar film contact to the major surface of the glass platform (2).

8. A device according to Claim 4, **characterised by** the contacts (7,7') being etched onto or into the major surface of the glass platform (2).

9. A device according to any of Claims 3 to 8, **characterised by** the major planar surface being an upper, in use, surface of the platform (2).

10. A device according to any preceding claim, **characterised by** the contacts (7,7') being disposed so as to provide a desired array whereby to be contactable by toe and heel regions of the feet of a user in use.

11. A personal body fat monitoring device according to any preceding claim, **Characterised by** also being a personal weighing scale.

## Patentansprüche

1. Körperfettüberwachungsgerät, das mindestens aufweist: mindestens ein Hauptflächenelement (2); elektrische Kontakte (7, 7'), die für eine Berührung durch die Füße eines Benutzers freigelegt sind; und eine elektronische Einrichtung (6') für das Berechnen des Körperfettes, die durch die Kontakte (7, 7') betätigt wird, **dadurch gekennzeichnet, daß** die Anordnung so ist, daß, wenn die elektrischen Kontakte berührt werden, die elektronische Einrichtung so aktiviert wird, daß das Körperfett eines Benutzers bestimmt wird.

2. Gerät nach Anspruch 1, **dadurch gekennzeichnet, daß** das Hauptflächenelement (2) eine Gewichtsaufnahmeplattform des Gerätes (1) aufweist.

3. Gerät nach Anspruch 2, **dadurch gekennzeichnet, daß** die Plattform (2) ein Glas aufweist, von dem eine obere benutzte ebene Hauptfläche die Kontakte (7, 7') hält.

4. Gerät nach Anspruch 3, **dadurch gekennzeichnet, daß** das Glas eine Glasscheibe (2) per se aufweist.

5. Gerät nach Anspruch 4, **dadurch gekennzeichnet, daß** die Kontakte (7, 7') mechanisch an der Hauptfläche der Glasplattform gesichert werden.

6. Gerät nach Anspruch 4, **dadurch gekennzeichnet, daß** die Kontakte (7, 7') mittels eines Klebstoffes an der Hauptfläche der Glasplattform (2) gesichert werden.

7. Gerät nach Anspruch 4, **dadurch gekennzeichnet, daß** die Kontakte (7, 7') durch Galvanisierung oder einen ähnlichen Filmkontakt auf der Hauptfläche der Glasplattform (2) gesichert werden.

8. Gerät nach Anspruch 4, **dadurch gekennzeichnet, daß** die Kontakte (7, 7') auf oder in die Hauptfläche der Glasplattform (2) geätzt werden.

9. Gerät nach einem der Ansprüche 3 bis 8, **dadurch gekennzeichnet, daß** die ebene Hauptfläche bei Benutzung eine obere Fläche der Plattform (2) ist.

10. Gerät nach einem der vorhergehenden Ansprüche, **dadurch gekennzeichnet, daß** die Kontakte (7, 7') so angeordnet werden, daß sie eine gewünschte Anordnung liefern, die durch die Zehen- und Fersenbereiche der Füße eines Benutzers bei Benutzung berührt werden können.

11. Körperfettüberwachungsgerät nach einem der vorhergehenden Ansprüche, **dadurch gekennzeichnet, daß** es ebenfalls eine Personenwaage ist.

## Revendications

1. Dispositif de surveillance de la graisse corporelle, comprenant au moins un élément de surface majeur (2), des contacts électriques (7, 7') exposés en vue d'un contact avec les pieds d'un utilisateur, un moyen électronique (6') pour calculer la graisse corporelle, actionné par les contacts (7, 7'), **caractérisé en ce que** l'agencement est tel que la mise en contact des contacts électriques entraîne l'actionnement du moyen électronique pour déterminer la graisse corporelle d'un utilisateur.

2. Dispositif selon la revendication 1, **caractérisé en ce que** l'élément de surface majeur (2) comprend une plate-forme de réception du poids du dispositif (1).

3. Dispositif selon la revendication 2, **caractérisé en ce que** la plate-forme (2) comprend du verre dont une surface majeure plane, supérieure retient en service les contacts (7, 7').

4. Dispositif selon la revendication 3, **caractérisé en ce que** le verre comprend une feuille de verre (2) per se.

5. Dispositif selon la revendication 4, **caractérisé en ce que** les contacts (7, 7') sont fixés de manière mécanique sur la surface majeure de la plate-forme en verre.

6. Dispositif selon la revendication 4, **caractérisé en ce que** les contacts (7, 7') sont fixés par un adhésif sur la surface majeure de la plate-forme en verre (2).

7. Dispositif selon la revendication 4, **caractérisé en ce que** les contacts (7, 7') sont fixés par électrodéposition ou un contact par film similaire sur la surface majeure de la plate-forme en verre (2).

8. Dispositif selon la revendication 4, **caractérisé en ce que** les contacts (7, 7') sont gravés sur ou dans la surface majeure de la plate-forme en verre (2).

9. Dispositif selon l'une quelconque des revendications 3 à 8, **caractérisé en ce que** la surface plane majeure constitue en service une surface supérieure de la plate-forme (2).

10. Dispositif selon l'une quelconque des revendications précédentes, **caractérisé en ce que** les contacts (7, 7') sont agencés de sorte à établir un réseau voulu, pouvant être contacté en service par l'orteil et le talon du pied d'un utilisateur.

11. Dispositif de surveillance de la graisse corporelle selon l'une quelconque des revendications précédentes, **caractérisé en ce qu'**il constitue également un pèse-personne.
